# EUROPEAN PATENT SPECIFICATION

(11) **EP 3 558 397 B1**
(45) Date of publication and mention of the grant of the patent: **30.12.2020**
(21) Application number: 17826614.4
(22) Date of filing: 22.12.2017
(51) Int. Cl.: A61K 51/12, A61K 51/06

(54) **USE OF A BODY COMPRISING AN OXIDE OF LANTHANIDE SUPPORTED ON A SULPHUR CONTAINING CARBON BASED PARTICLE IN THERAPEUTIC APPLICATIONS**
VERWENDUNG EINES KÖRPERS, DER EIN LANTHANIDOXID UMFASST, DAS AUF EINEM SCHWEFELHALTIGEN KOHLENSTOFFBASIERTEN TEILCHEN GETRÄGERT IST, IN THERAPEUTISCHEN ANWENDUNGEN
UTILISATION D'UN CORPS, COMPRENANT UN OXYDE DE LANTHANIDE SUPPORTÉ SUR UNE PARTICULE À BASE DE CARBONE CONTENANT DU SOUFRE, DANS DES APPLICATIONS THÉRAPEUTIQUES

(30) Priority: 23.12.2016 NL 2018055
(43) Date of publication of application: 30.10.2019
(73) Proprietor: QUIREM Medical B.V., 7418 AH Deventer (NL)
(72) Inventor: NIJSEN, Johannes Franciscus Wilhelmus, 7418 AH Deventer (NL); BRANDTS, Jim Aloysius Maria, 3454 PK De Meern (NL); WAGEMAKER, Lorianne Irene, 3454 PK De Meern (NL); BERBEN, Pieter Hildegardus, 3454 PK De Meern (NL); SCHLESINGER, Malk, 83308 Trostberg (DE)
(74) Representative: V.O.
(86) International application number: PCT/NL2017/050878
(87) International publication number: WO 2018/117847

(56) References cited:
- WO-A1-02/34300
- WO-A1-2013/144879

## Description

The invention is directed to the use of a body comprising oxides of lanthanides, in particular holmium oxide (Ho₂O₃), which are supported on a sulphur containing carbon based particle in therapeutic applications.

Lanthanides, particularly holmium, can be used in the treatment, in particular by radiotherapy, of various forms of cancers and tumours, such as those which can be found in the liver and the brain. Upon neutron irradiation ¹⁶⁵Ho is converted to the radioactive isotope ¹⁶⁶Ho, which is a beta-rachation emitter. Lee et al., European Journal of Nuclear Medicine 2002, 29(2), 221-230 has shown that the radio-active holmium can be effective in radioablation treatment of malignant melanoma in a rat model.

Holmium is particularly attractive since it is both a beta- and gamma-emitter when irradiated to Holmium-166 (¹⁶⁶Ho). Consequently, it can be used in both nuclear imaging and radioablation. Further, it is known in the art that holmium can be visualised by computer tomography and MRI due to its high attenuation coefficient and paramagnetic properties, as described for instance by Bult et al., Pharmaceutical. Research 2009, 26(6), 1371-1378.

Various attempts have been made to locally administer radionuclides, such as radioactive isotopes of lanthanides, particularly holmium, as a treatment for cancer with mixed results.

WO-A-02/34300 describes a particulate material comprising a polymer matrix, in particular an ion exchange resin, and a radionuclide, a method for the preparation thereof and use of this particulate material. WO-A-02/34300 describes preparing this particulate material by adsorbing a radionuclide onto a polymer matrix and precipitating the radionuclide as an insoluble salt to stably incorporate the radionuclide into the polymer matrix. The disadvantage of this particulate material is that leaching of the radionuclide from the particulate material occurs upon contact with aqueous solutions at neutral pH and to a greater extent at acidic pH, which results in inappropriate radiation of other tissues and complications due to toxicity of the leached components of the particulate material, including the radionuclide, non-radioactive components and elements resulting from the radioactive decay of the radionuclide.

WO-A-2013/144879 describes bodies comprising amorphous carbon supported nanoparticles comprising oxides of lanthanides, a method of preparation thereof and the use of said bodies in therapeutic applications. WO-A-2013/144879 describes that the bodies are prepared by impregnating a carbon source material by contacting it with an aqueous solution of a salt of a lanthanide, drying the impregnated material and subjecting the dried impregnated material to pyrolysis under inert conditions. Although this material is an improvement over known products in reducing the problem of leaching of the radionuclide upon contact with aqueous solutions, in particular at neutral pH, there is still a need for further reducing the leaching of radionuclides from particulate materials in solutions of low (acidic) pH.

WO-A-2009/011589 describes holmium acetylacetonate (HoAcAc) microspheres and the preparation thereof.

It is an object of the invention to provide comprising particles of one or more oxides of lanthanides, in particular holmium, on a particulate support with improved properties over known materials for use in medical applications, in particular with respect to improving the stability of the lanthanide oxides in a carrier material in a liquid, such as an aqueous solution or a biological fluid (*e.g*. blood), especially under neutral and acidic conditions.

It was found that this object can be realised by using a body comprising an oxide of lanthanide, wherein preferably the oxide of lanthanide is in the form of nanoparticles, supported on a sulphur containing carbon based particle as a medicament or in a method of surgery, therapy and/or *in vivo* diagnostics.

Accordingly, a first aspect the invention is directed to a body comprising an oxide of lanthanide supported on a sulphur containing carbon based particle, wherein said body comprises lanthanide to sulphur in an atomic ratio ranging from 1.00 : 0.01 to 1 : 10 for use as a medicament (*i.e.* pharmaceutical in a method for the treatment of the human or animal body) and/or a diagnostic agent. The invention is in particular directed to the said body for use as a medicament, or for use in (a method of) surgery, therapy and/or *in vivo* diagnostics (of the human or animal body).

When referring to a noun (*e.g.* a body, a salt, an element, *etc*.) in the singular, the plural is meant to be included, or it follows from the context that it should refer to the singular only.

The term "substantially) or "essentially)" is generally used herein to indicate that it has the general character or function of that which is specified. When referring to a quantifiable feature, these terms are in particular used to indicate that it is for at least 75 %, more in particular at least 90 %, even more in particular at least 95 % of the maximum that feature.

The term "low pH" as used herein is defined as an "acidic pH", *i.e.* the pH is less than 7.

The term "room temperature", as used herein, is defined as a temperature of about 25 °C.

The term "cancer", as used herein, refers to a malignancy, such as a malignant tumour, which is typically a solid mass of tissue that is present (*e.g.* in an organ or the lymph system) of the human or animal body. The terms "cancer" and "tumour" are used interchangeably herein.

The term "individual", as used herein, is defined herein as "the human or animal body".

The terms "treatment" and "treating" as used herein are not meant to be limited to curing. Treating is meant to also include alleviating at least one symptom of a disease, and/or delaying the course of a disease.

In a further aspect, the invention is directed to a body comprising an oxide of lanthanide, supported on a sulphur containing carbon based particle, wherein said body comprises lanthanide to sulphur in an atomic ratio ranging from 1 : 0.01 to 1 : 10 for use as a medicament for treating a cancer, in particular a cancer of the brain, pancreas, lymph, lung, head and neck, prostate, breast, liver, intestines, thyroid, stomach, or kidney.

In yet a further aspect, the invention is directed to a body comprising an oxide of lanthanide, supported on a sulphur containing carbon based particle, wherein said body comprises lanthanide to sulphur in an atomic ratio ranging from 1 : 0.01 to 1 : 10 for use as a diagnostic agent for detecting a cancer, in particular a cancer of the brain, pancreas, lymph, lung, head and neck, prostate, breast, liver, intestines, thyroid, stomach, or kidney.

In a preferred embodiment, the body is used according to the invention as a medicament (such as a pharmaceutical). In particular, the body is used in the preparation of a pharmaceutical (preferably for the treatment of a medical disorder (*i.e.* disease/condition, such as cancer).

Preferably, said body is used (preferably as a medicament) in a method according to the invention for the treatment of the human or animal body.

More preferably, said treatment is a method of surgery, therapy and/or *in vivo* diagnostics. More in particular, the method of surgery, therapy and/or *in vivo* diagnostics comprises:
- imaging, imaging, such as magnetic resonance imaging, nuclear scanning imaging, X-ray imaging, positron emission tomography (PET) imaging, single-photon emission computed tomography (SPECT) imaging, X-ray computed tomography (CT) imaging, scintigraphy imaging, ultrasound, and/or fluorescent imaging;
- drug delivery;
- cellular labeling; and/or.
- radiotherapy.

The body used according to the invention is capable of at least in part in disturbing a magnetic field. Said body can be detected by a non-radioactive scanning method such as magnetic resonance imaging (MRI).

Preferably, said body is in the form of a suspension. The meaning of the word suspension, as used herein, should also be understood as at least including dispersions. Typically, the suspension comprises the body and a (carrier) fluid or gel. Suitable (carrier) fluids which may be used in said suspension include aqueous solutions, such as a saline solution (*i.e.* sodium chloride in water), a phosphate buffered saline (PBS) solution, or a tris buffered saline solution. Optionally, said aqueous solution also comprises pluronic and/or polysorbates 20 or 80 (*i.e.* TWEEN 20 or TWEEN 80). A suitable gel for use in said suspension is a dextran or gelatin starch or hyaluronic acid, *etc.*

Accordingly, the invention is also directed to a suspension comprising a number of bodies as described herein, said suspension being a therapeutic suspension, an MRI scanning suspension, and/or a nuclear scanning suspension.

More in particular, the invention relates to a contrast agent comprising a number of bodies as described herein. Such a contrast agent can, for instance, be used for inflammation and/or lymph node detection. The contrast agent may, for instance, be a blood pool contrast agent for computer tomogram (CT) including dual energy CT, and magnetic resonance imaging (MRI).

The body used according to the invention may be administered as a medicament; or, in a method of surgery, therapy and/or *in vivo* diagnostics by suitable means, such as by catheter (for example radioembolisation of liver tumours), (direct or intravenous) injection, infusion, a patch on the skin of an individual (*i.e.* a skin patch), *etc.*

Magnetic resonance imaging (MRI) provides information of the internal status of an individual. A contrast agent is often used in order to be capable of obtaining a scanning image. For instance iron and gadolinium, preferably in the form of ferrite particles and gadolinium -DTPA (diethylaminetriaminepentaacetic acid) complexes, are often used in contrast agents for MRI scanning. This way, a good impression can be obtained of internal disorders, like the presence of (a) tumour(s). After diagnosis, a treatment is often started involving administration of a pharmaceutical composition to an individual. It is often important to monitor the status of the individual during treatment as well. For instance the course of a treatment and targeting of a drug can be monitored, as well as possible side effects which may imply a need for terminating, or temporarily interrupting, a certain treatment.

In a further preferred embodiment, the body is used according to the invention in a method for detecting cancer, which method comprises the steps of:
- administering a body (*i.e.* a body comprising an oxide of lanthanide, wherein preferably the oxide of lanthanide is in the form of particles (such as nanoparticles), supported on a sulphur containing carbon based particle, wherein said body comprises lanthanide to sulphur in an atomic ratio ranging from 1 : 0.01 to 1 : 10) to an individual;
- obtaining a scanning image; and
- determining whether said image reveals the presence of a cancer.

The body used according to the invention in said method for detecting cancer may suitably be administered in the form of a suspension, as described herein above.

Sometimes local treatment in only a specific part of the individual is preferred. For instance, tumour growth can sometimes be counteracted by a method of internal radiotherapy comprising administration of a radioactive body (*i.e.* a radioactive body comprising an oxide of lanthanide, wherein preferably the oxide of lanthanide is in the form of (nano)particles, supported on a sulphur containing carbon based particle, wherein said body comprises lanthanide to sulphur in an atomic ratio ranging from 1 : 0.01 to 1 : 10 and comprises a radioactive isotope of said lanthanide) used in the method of the invention to an individual. If said radioactive body accumulates inside and/or around the tumour, specific local treatment is possible.

In a particular embodiment, the body, when administered intravenously via an injection in the blood vessel and which accumulates in the cancer due to the enhanced permeability and retention (EPR) effect, typically has a diameter in the range of less than 10 µm, preferably from 0.01 to 0.50 µm, and more preferably 0.05 to 0.30 µm.

The body, when administered to a cancer via a catheter, typically has a diameter in the range of 1-400 µm, preferably 1-200 µm, more preferably 1-100 µm and even more preferably 15-60 µm. The body, when administered to a cancer via a direct injection (*i.e.* intratumoural injection), typically has a diameter in the range of 1 to 100 µm, preferably 1-50 µm, more preferably 1-30 µm and even more preferably 5-20 µm. Such bodies can attractively be used for local therapeutic and in addition diagnostic purposes. For local therapeutic purposes the body(ies) can suitably be delivered (*i.e.* administered) locally via a catheter or via direct injection, whereas for diagnostic purposes the body(ies) can be introduced (*i.e.* administered) into a human or animal body via parenteral administration, *e.g.* via injection, infusion, *etc.*

In another particular embodiment, the body is used according to the invention in a method for treating cancer, which method comprises the steps of:
- administering a body (*i.e.* a body comprising an oxide of lanthanide, wherein preferably the oxide of lanthanide is in the form of (nano)particles, supported on a sulphur containing carbon based particle, wherein said body comprises lanthanide to sulphur in an atomic ratio ranging from 1 : 0.01 to 1 : 5) to an individual;
- administering to the individual a therapeutic composition comprising said body.

Preferably, the method for treating cancer comprises the steps of:
- administering a body (*i.e.* a body comprising an oxide of lanthanide, wherein preferably the oxide of lanthanide is in the form of (nano)particles, supported on a sulphur containing carbon based particle, wherein said body comprises lanthanide to sulphur in an atomic ratio ranging from 1 : 0.01 to 1 : 5) to an individual;
- obtaining a scanning image of the individual; and
- determining the distribution of said body within the individual; and,
- administering to the individual a therapeutic composition comprising said body.

In yet a further aspect, the invention is directed to the use of a body comprising an oxide of lanthanide, supported on a sulphur containing carbon based particle, wherein said body comprises lanthanide to sulphur in an atomic ratio ranging from 1 : 0.01 to 1 : 10 for the preparation of a medicament for treating a cancer, in particular a cancer of the brain, pancreas, lymph, lung, head and neck, prostate, breast, liver, intestines, thyroid, stomach, or kidney.

In yet a further aspect, the invention is directed to a pharmaceutical composition comprising
- a body comprising an oxide of lanthanide, supported on a sulphur containing carbon based particle, wherein said body comprises lanthanide to sulphur in an atomic ratio ranging from 1 : 0.01 to 1 : 10, and
- a pharmaceutically acceptable carrier, diluent and/or excipient.

The body in said therapeutic composition is typically radioactive and /or is provided with at least one active group. Preferably, the body and the therapeutic composition suitable for use in said method of treating cancer are each in the form of a suspension, as described herein above.

Preferably, the form of radiotherapy used is radio-embolisation. Radio-embolisation is a treatment which combines radiotherapy with embolisation. Typically, the treatment comprises administering (*i.e.* delivering) the body used according to the invention, for instance via catheterisation, into the arterial blood supply of an organ to be treated, whereby said body becomes entrapped in the small blood vessels of the target organ and irradiate the organ. In an alternate form of administration the body may be injected directly into a target organ or a solid tumour to be treated (*i.e.* intratumoural injection). The person skilled in the art, however, will appreciate that the administration of the body used according to the method of the invention may be by any suitable means and preferably by delivery to the relevant artery. The body may be administered by single or multiple doses, until the desired level of radiation is reached. Preferably, the body is administered as a suspension, as described herein above.

In a preferred embodiment, the method of surgery, therapy and/or *in vivo* diagnostics is a method of detecting and/or treating a cancer, but particularly in the treatment of brain, pancreas, lymph, lung, head and neck, prostate, intestines, thyroid, stomach, breast, liver and kidney cancers, and more in particular metastases, by administering said body. Said body may suitably be administered to cancers of the brain, pancreas, intestines, thyroid, stomach, head and neck, lung and breast cancers and tumours via an (intratumoural) injection. Said body may also be suitably administered to cancers of the liver, kidney, pancreas, brain, lung and breast via a catheter.

The body used according to the invention in said method of detecting and/or treating of a cancer, typically tends to accumulate in cancer tissue substantially more than it does in normal tissues due to the enhanced permeability and retention (EPR) effect, particularly when the body has a size of 0.01 to 2 µm and more in particular 0.01 to 0.9 µm. It is believed that this phenomenon is a consequence of the rapid growth of cancer cells, which stimulates the production of blood vessels. Typically, vascular endothelial growth factor (VEGF) and other growth factors play a role in cancer angiogenesis. Tumour cell aggregates (*i.e.* tissues) having a size of 1-2 mm usually start to become dependent on blood supply carried out by neovasculature for their nutritional and oxygen supply. These newly formed tumour vessels are usually abnormal in form and architecture. The tumour cells are poorly aligned defective endothelial cells with wide fenestrations, lacking a smooth muscle layer, or innervation with a wider lumen, and impaired functional receptors for angiotensin II. Furthermore, the tumour tissues usually lack effective lymphatic drainage. All of these factors lead to abnormal molecular and fluid transport dynamics. The EPR effect is further enhanced by many pathophysiological factors involved in enhancement of the extravasation of the body in solid tumour tissues. One factor that lends to the increased retention is the lack of lymphatics around the tumour region which would filter out such particles (*i.e.* the body) under normal conditions. The EPR effect helps to carry the body and spread it inside the cancer tissue (*i.e.* solid tumours) enabling it to be more effective in methods of detecting and/or treating cancers.

In yet a further aspect, the invention is directed to the body as described herein for use in a method for the treatment of a tumour in an individual, wherein the dosage of said bodies is derived from a scanning image obtained with a scanning suspension comprising particles capable of at least in part disturbing a magnetic field, preferably with a diameter of at least 1 µm, and with the same chemical structure as the bodies, based on the distribution of the particles with the same chemical structure within said individual, and wherein said bodies for use in the method for the treatment of said tumour are more radioactive than said particles used for obtaining said scanning image.

Preferably, the body comprises lanthanide to sulphur in an atomic ratio of at least 1.0 : 0.1 and more preferably at least 1.0 : 0.4. Preferably, the body comprises lanthanide to sulphur in an atomic ratio of at most 1 : 5 and more preferably at most 1 : 3.5.

The body is a composite material typically comprising lanthanide and sulphur in a total amount of at least 5 wt.%, preferably at least 10 wt.%, and more preferably at least 20 wt.%, even more preferably at least 30 wt.%, in particular at least 40 wt.%, and even more in particular at least 50 wt.%, calculated as the total amount of elemental lanthanide and elemental sulphur based on the weight of said body. As an upper limit, the body typically comprises lanthanide and sulphur in a total amount of at most 90 wt.%), preferably at most 80 wt.%, even more preferably at most 70 wt.%, in particular at most 60 wt.%, more in particular at most 50 wt.%, and even more in particular 40 wt.%, calculated as the total amount of elemental lanthanide and elemental sulphur based on the weight of said body.

Typically, the body comprises elemental carbon in an amount of 5-80 wt.%, preferably 10-80 wt.%, more preferably 20-70 wt.% and even more preferably 40-70 wt.%, based on the weight of said body. Preferably, the elemental carbon present in said body is in the form of amorphous carbon, graphitic carbon and combinations therefore, and preferably amorphous carbon.

The carbon based particle of the body, in addition to comprising elemental carbon, also typically comprises a carbon source material. The carbon source material is preferably functionalised with at least one sulphur (containing) group selected from the list consisting of sulphonic acid, sulphoxide, sulphate, sulphite, sulphone, sulphinic acid, thiol, thioether, thioester, thioacetal, thione, thiophene, thial, sulphide, disulphide, polysulphide and sulphoalkyl (e.g. sulphobutyl) groups, and combinations thereof, and more preferably a sulphonic acid group.

The carbon source material may be a polymeric matrix, wherein preferably said polymeric matrix is partially cross-linked, in particular cross-linked in an amount of 1-20 %, and more in particular cross linked in an amount of 2-10 %. In a particular embodiment, the polymeric matrix is an ion exchange resin, preferably a cation exchange resin, and more preferably a cation exchange resin comprising an aliphatic polymer, such as polystyrene. One particularly preferred cation exchange resin is a styrene/divinylbenzene copolymer resin.

The carbon source material may also be a material selected from the group consisting of cellulose, such as microcrystalline (MCC); cellulose-like material, such as cotton; carbohydrate, such as sugar or chitosan; active carbon; and, combinations thereof.

The advantage of the use of the body according to the invention is that the presence of sulphur in the carbon based particle (*i.e.* support/carrier) is that it enhances the stability of the lanthanide oxides in the body, particularly in acidic conditions. Without wishing to be bound to by theory, it is believed that sulphur in the body (*e.g*. sulphate or sulphoxide) is at least partly responsible for a good distribution of the holmium throughout the body. In addition, the presence of thiophenic (and sulphite) functionalities as a result of the heat treatment, will enhance the incorporation of the lanthanide oxides into the body. This effectively prevents or substantially limits the leaching out (extraction) of the lanthanide from the body in a liquid, such as an aqueous solution or a biological fluid (*e.g*. blood), in particular at low pH or in the presence of cations and anions.

Another advantage of using the body according to the invention is that the carbon in the carbon based carrier functions as a neutron moderator, which is relatively stable against neutron irradiation. Carbon also is typically resistant to modification of its shape (*i.e.* keeps it shape) and substantially chemically inert. Further, the surface of the carbon may be functionalised according to known methods in the art.

In a specific embodiment, the lanthanide at least partially comprises a radioactive isotope of said lanthanide. The radioactive isotope of said lanthanide may be generated by numerous methods, a non-exhaustive list includes neutron irradiation, laser pulse generation, laser-plasma interaction, cyclotron and using other sources of neutrons. For example, upon neutron irradiation ¹⁶⁵Ho is converted to ¹⁶⁶Ho. The body may suitably be a radioactive body. Preferably, however, the body is initially non-radioactive (*i.e.* prior to use in a medical application), which has the advantage in that it avoids personnel being exposed to radiation and the need for specially equipped facilities, such as hot cells and transport facilities.

The lanthanide of the body is selected from the series of lanthanide elements, which comprises the fifteen metallic chemical elements with atomic numbers from 57 to 71, *i.e.* the group consisting of La (atomic number 57), Ce (58), Pr (59), Nd (60), Pm (61), Sm (62), Eu (63), Gd (64), Tb (65), Dy (66), Ho (67), Er (68), Tm (69), Yb (70), Lu (71) and combinations thereof (lanthanum, cerium, praseodymium, neodymium, promethium, samarium, europium, gadolinium, terbium, dysprosium, holmium, erbium, thulium, ytterbium, and lutetium). Preferably, the lanthanide is one or more selected from the group consisting of lanthanum, neodymium, gadolinium, dysprosium, and holmium. More preferably, the lanthanide is holmium.

Typically, the oxide of lanthanide is in the form of particles, and preferably nanoparticles. The nanoparticles of the oxide of lanthanide more preferably have a diameter of 10 nm or less, and even more preferably have a diameter which is 5 nm or less. The diameter of the nanoparticles, as used herein, is typically the value that can be determined by X-ray Diffraction, unless otherwise indicated. Typically, the diameter of the nanoparticle is calculated from the peak width of the diffraction pattern of a specific component using the Scherrer equation The diameter of the nanoparticles may also be suitably determined by transmission electron microscopy (TEM) or scanning electron microscopy (SEM). The lanthanide oxide (nano)particles are typically present on the surface of the sulphur containing carbon based particle, which surface includes the (inner) pore area.

The body can be tailored to any desired size from several tens of nanometers up to a millimeter dependent upon its intended medical application. Typically, the size of the body (*i.e.* diameter) is at least 0.01 µm, preferably at least 0.05 µm, more preferably at least 0.1 µm, even more preferably at least 1 µm and in particular at least 15 µm. The body also typically has a diameter of at most 500 µm, preferably at most 400 µm, more preferably at most 300 µm, even more preferably at most 200 µm, in particular at most 100 µm, and more in particular at most 60 µm. The size of the body, as used herein, is preferably the value as measured according to International Standard ISO 13319 on a Multisizer 3 Coulter Counter, Beckman Coulter, equipped with a 100 µm orifice.

The diameter of the body typically refers to a spherical particle. In case the shape of the body deviates from spherical, the diameter refers to the largest dimension of the particle. Preferably the body is spherical or essentially spherical, in particular having a sphericity of close to 1, for instance more than 0.75, preferably more than 0.85 and more preferably at least 0.95. The sphericity of a certain particle is the ratio of the surface area of a sphere having the same volume as said particle to the surface area of said particle.

Although the lanthanide oxide particles in the body are very small, the crystal structure is the same as the normally occurring crystal structure of the bulk oxide material, which is cubic for all oxides of lanthanides.

Typically, the body has a density which is tailored to the intended use. Typically, when used in medical applications, in particular in radiotherapy, the body has a density of > 0.8 g/ml to 8.0 g/ml, preferably about 0.9-6.0 g/ml, more preferably 1.0-4.0 g/ml, and even more preferably 1.0-3.5 g/ml, in particular 1.05-2.00 g/ml, and more in particular 1.1-1.6 g/ml. The advantage of such a low density for the body is that this makes it compatible with the density of biological fluids, such as a blood stream. This in turn leads to a substantially homogeneous distribution of the body within a target organ, which prevents or substantially minimises the occurrence of focal areas of excessive radiation.

In a preferred embodiment, the body comprises one or more active and/or hydrophilic groups (chemically) attached to/are present on the surface of the body. Such hydrophilic groups may be selected from sulphonic acid groups, hydroxyl groups, carbonyl groups, carboxyl groups, sulphhydryl groups, amino groups, polyaromatic groups and combinations thereof, preferably hydroxyl, carboxyl and/or sulphonic acid groups. Such active groups may be selected from antibodies, nucleic acids, lipids, fatty acids, carbohydrates, polypeptides, amino acids, proteins, plasma, antigens, liposomes, hormones, markers and combinations thereof.

In another preferred embodiment, the body, in particular the oxide of lanthanide of said body, is at least partly, preferably completely, coated by a layer of an element or an element oxide (*i.e.* an oxide of an element), wherein said element is selected from the group consisting of silicon, titanium, zirconium, hafnium, cerium, aluminium, niobium, tantalum and combinations thereof. More preferably, said layer coating of said body is of a substantially uniform thickness. The advantage of the body further comprising such a coating when used in a medical application is that it can provide stability against leaching of components (*e.g*. radionuclides, elements, *etc.*) from the body, influences the hydrophilicity and/or enables tuning of the density of said body, particularly when used in combination with selective oxidation (*i.e.* carbon is at least partly removed) of the coated body. Selective oxidation may also be applied to the uncoated body; or, to the coated or uncoated carbon source material (optionally further comprising a salt of a lanthanide) prior to pyrolysis.

In a further preferred embodiment, the body also comprises at least one element selected from the group consisting of iron, gadolinium, manganese, phosphorous, iodine, iridium, rhenium and combinations thereof. The advantage of the body further comprising iron manganese and/or gadolinium is that these elements are paramagnetic, which enhances the therapeutic and diagnostic effectiveness of the body when used as a medicament in surgery or therapy and diagnostic methods. The advantage of using phosphorous, iodine, iridium and/or rhenium is that the radionuclides of these elements have a radioactive decay particularly suitable for medical applications.

The body used according to the invention may be prepared by a process comprising the steps of:
- contacting a carbon source material, wherein preferably said carbon source material comprises at least one sulphur group, with an aqueous solution of a salt of a lanthanide thereby producing a modified carbon source material;
- drying the modified carbon source material; and,
- subjecting said dried modified carbon source material to pyrolysis under inert conditions.

In a preferred embodiment, the process for preparing the body comprises introducing lanthanide cations into the carbon source material, wherein preferably said carbon source material comprises at least one sulphur group, by ion exchange by contacting it with the aqueous solution of the salt of said lanthanide, thereby producing a modified carbon source material.

In another preferred embodiment, the process for preparing the body comprises impregnating the carbon source material, wherein preferably said carbon source material comprises at least one sulphur group, with the salt of said lanthanide by contacting it with the aqueous solution of the salt of said lanthanide, thereby producing the modified (*i.e.* impregnated) carbon source material. The impregnation method suitable to be used may be incipient wetness, wet impregnation or vacuum impregnation.

Optionally, the modified carbon source material is washed in a washing step with a liquid prior to the pyrolysis step. Suitable liquids may include water and/or an alcohol, such as isopropanol. The advantage of carrying out this washing step is that it removes any residual salts (*i.e.* non-ion exchanged holmium salts) and other components, such as HNO₃, which may be present in the modified carbon source material.

In a further preferred embodiment, the process for preparing the body comprises depositing lanthanide by precipitation onto the carbon source material, wherein preferably said carbon source material comprises at least one sulphur group, by contacting it with the aqueous solution of the salt of said lanthanide, thereby producing the modified carbon source material.

A suitable carbon source material for use in the process for preparing the body may be a polymeric matrix, wherein preferably said polymeric matrix is partially cross-linked, in particular cross-linked in an amount of 1-20 %, and more in particular cross linked in an amount of 2-10 %. In a particular embodiment, the polymeric matrix is an ion exchange resin, preferably a cation exchange resin, and more preferably a cation exchange resin comprising an aliphatic polymer, such as polystyrene. One particularly preferred cation exchange resin is a styrene/divinylbenzene copolymer resin.

The carbon source material suitable for use in the process for preparing the body may also be selected from the group consisting of cellulose, such as microcrystalline (MCC); cellulose-like material, such as cotton; carbohydrate, such as sugar or chitosan; active carbon; and, combinations thereof.

Preferably, the carbon source material comprises at least one sulphur (containing) group on the surface of said carbon source material, and more preferably the sulphur (containing) group is selected from the list consisting of sulphonic acid, sulphoxide, sulphate, sulphite, sulphone, sulphinic acid, thiol, thioether, thioester, thioacetal, thione, thiophene, thial, sulphide, disulphide, polysulphide and sulphoalkyl (e.g. sulphobutyl) groups, and combinations thereof, even more preferably a sulphonic acid group.

The carbon source material may optionally be pre-treated prior to use in the method for preparing a body by rinsing with water of an organic solvent, such as an alcohol or acetone. Another optional pre-treatment may be contacting via ion exchange the carbon source material with an aqueous solution comprising at least one soluble salt, thereby replacing at least part of any cations present in the carbon source material. Suitable soluble salts which could be used include ammonium chloride or sodium chloride. It is believed that such a pre-treatment could influence the distribution of the lanthanide oxides in the body and/or the porosity/density of the resulting body.

Suitable lanthanide salts which may be used in the process for preparing the body are soluble lanthanide salts including nitrate, chloride, phosphate and/or organic salts (*e.g.* acetate and citrate). Preferably, the lanthanide salt is lanthanide nitrate.

Preferably, the contacting step of the process for preparing the body is carried out at a temperature of between 15 °C and 100 °C, more preferably between 20 °C and 60 °C.

Typically the drying step is carried out until the dried product reaches constant weight. Preferably the drying is carried out at least at room temperature, and more preferably at a temperature of between 80 and 150 °C. The drying step may be carried out in more than one step.

The pyrolysis step temperatures may be carried out at a temperature of at least 200 °C, preferably at least 300 °C, more preferably at least 600 °C, even more preferably at least 700 °C, and in particular at least 800 °C. The maximum temperature suitable to use for the pyrolysis step is typically at most 2000 °C, preferably at most 1500 °C, and more preferably at most 1000 °C. This step is carried out under inert conditions, *viz*. under conditions that avoid reaction of carbon with the surroundings. Preferably these conditions comprise exclusion of oxygen from air. This may preferably be obtained by carrying out the pyrolysis under a typical "inert" gas, such as nitrogen or a noble gas, such as argon or helium, which is used to dissipate the oxygen containing air.

During the pyrolysis of the carbon source material the size of the particles is decreased. Typically, the size of the carbon source material is reduced by 5-50 % in diameter. More typically between 10-40 %. Precise control of the diameter of the particles is possible by controlling of the pyrolysis conditions (*i.e.* temperature, duration and gas composition).

Optionally, the pyrolysed modified carbon source material is subjected to a post treatment. One suitable post treatment comprises the step of mixing the pyrolysed modified carbon source material with an excess of liquid (*i.e.* water) comprising a surfactant to form a suspension, subjecting the suspension to agitation, filtering the suspension, washing and then drying the post treated pyrolysed modified carbon source material. Suitable surfactants which may be used include non-ionic and/or anionic surfactants. The agitation step may be carried out by using a stirrer or a mixer, such as an ultrasonic mixer. The washing and drying steps are as described herein above. The advantage of this step is that removes any water soluble byproducts formed during the pyrolysis step and helps to take apart assembled particles into individual particles without damaging the surface.

In a preferred embodiment, the process for preparing the body further comprises loading the carbon source material with a precursor of other elements selected from the group consisting of iron, gadolinium, manganese, phosphorous, iodine, iridium, rhenium and combinations thereof. The precursor of these other elements may be loaded by methods known in the art, such as, ion exchange, impregnation and/or deposition precipitation.

Preferably, the process for preparing the body comprises an additional step in which the body is functionalised by attaching one or more active and/or hydrophilic groups to the surface of the sulphur containing carbon based particle. Since the surface of said particle typically comprises graphitic and/or amorphous carbon, attaching chemical groups to the surface is relatively easy using techniques known in the art. Such hydrophilic groups and active groups correspond to those groups as mentioned herein above.

In another preferred embodiment, the process for preparing the body further comprises at least partly, preferably completely, coating the carbon source material either prior to or after contacting the carbon source material with an aqueous solution of a salt of a lanthanide; or, the body, in particular the oxide of lanthanide of the body; by a layer of an element or element oxide (*i.e.* an oxide of an element). Suitable elements are selected from the group consisting of silicon, titanium, zirconium, hafnium, cerium, aluminium, niobium, tantalum and combinations thereof.

The layer of the element or element oxide may be applied to the carbon source material either prior to or after contacting the carbon source material with an aqueous solution of salt of a lanthanide; or, to the body, in particular the oxide of lanthanide of the body, by suitable means. One such suitable means includes using the sol-gel method. Suitable starting materials for use in the sol-gel method include alkoxides, chlorides or a stabilised sol of said elements (*i.e.* silicon, titanium, zirconium, hafnium, cerium, aluminium, niobium and/or tantalum). Typically, in the sol-gel method the pH is adjusted to either be more basic or acidic using compounds known in the art. Such suitable compounds include ammonia, aqueous ammonium, hydroxide solution, alkali hydroxides, fluoride salts or mineralic acids. More preferably, said layer coating of said body is of a substantially uniform thickness.

The process for preparing the body may include a following step in which carbon is at least partly removed (*i.e.* selective oxidation) from the coated or uncoated body; or, from the coated or uncoated carbon source material (optionally further comprising a salt of a lanthanide) prior to the pyrolysis step. Typically, the carbon may be removed in a calcination step, wherein the coated or uncoated body; or, the coated or uncoated carbon source material (optionally further comprising a salt of a lanthanide) prior to the pyrolysis step; is calcined in an oxygen containing gas flow at a temperature of 900 °C or less, preferably 600 °C or less, more preferably 500 °C or less, and even more preferably 400-500 °C.

The invention has been described by reference to various embodiments, and methods. The skilled person understands that features of various embodiments and methods can be combined with each other.

The term "or" as used herein is defined as "and/or" unless specified otherwise. The use of the terms "a" and "an" and "the" and similar referents in the context of describing the invention (especially in the context of the claims) are to be construed to cover both the singular and the plural, unless otherwise indicated herein or clearly contradicted by context. The terms "comprising", "having", "including" and "containing" are to be construed as open-ended terms (*i.e.,* meaning "including, but not limited to") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. For the purpose of the description and of the appended claims, except where otherwise indicated, all numbers expressing amounts, quantities, percentages, and so forth, are to be understood as being modified in all instances by the term "about". Also, all ranges include any combination of the maximum and minimum points disclosed and include any intermediate ranges therein, which may or may not be specifically enumerated herein.

Preferred embodiments of this invention are described herein. Variation of those preferred embodiments may become apparent to those of ordinary skill in the art upon reading the foregoing description. The inventors expect skilled artisans to employ such variations as appropriate, and the inventors intend for the invention to be practiced otherwise than as specifically described herein. Accordingly, this invention includes all modifications and equivalents of the subject-matter recited in the claims appended hereto as permitted by applicable law. Moreover, any combination of the above-described elements in all possible variations thereof is encompassed by the invention unless otherwise indicated herein or otherwise clearly contradicted by context. The claims are to be construed to include alternative embodiments to the extent permitted by the prior art.

The various aspects of the invention are now illustrated on the basis of the following non-limiting examples.

### Examples

### Example 1

30 grams of washed and dried ion exchange resin (Dowex 50WX4 H+ (*i.e.* a styrene-divinylbenzene polymer matrix functionalised with a sulphonic acid group), Fluka, 200-400 mesh) spherically shaped particles, having a diameter between 34 and 74 micron, was added to 330 grams demi-H₂O and stirred at 750 rpm in a glass beaker. To this slurry 17.5 g of holmium nitrate pentahydrate (Ho(NO₃)₃·5H₂O, Sigma-Aldrich, 99.9 % purity) was added and the mixture was stirred overnight, which resulted in the holmium cations being introduced into the ion exchange resin by ion exchange. Next, the slightly brownish particles were filtered and washed with 300 ml H₂O and 200 ml isopropanol (technical grade, VWR). Drying was performed in an oven with 18.1 g of the holmium loaded ion exchanged resin material under a nitrogen flow of 62 nl/h and was heated to 120 °C for 16 hours overnight. Subsequently, the material was heated to 200 °C with 2 °C/min for 2 hours while shaking the reactor several times to keep the material fluidised and dried to a constant weight. The dried material was then pyrolysed by heating to 800 °C (ramp 2 °C/min., hold 1 hour) under a nitrogen flow of 16.0 nl/h with fluidisation (*i.e.* the volume of the bed is increased with a factor 2-3 without blowing particles out of the reactor), which produced a black powder. After the pyrolysis step was completed, the pyrolysed material was allowed to cool to room temperature while being kept under a N₂ flow.

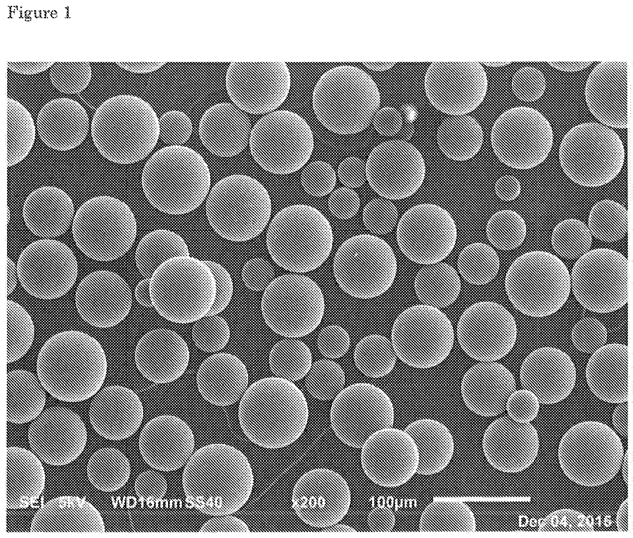

Finally the pyrolysed material was treated with an excess demi-water with a few droplets of surfactant (Dreft soap for dish washing, which comprises a mixture of 5-15 % of anionic surfactants and < 5 % nonionic surfactants). The suspension was treated in an ultrasonic bath for 60 minutes and subsequently filtered, washed with 500 ml of demi-water and finally with 100 ml *i*-propanol. The pyrolysed material was dried in an oven at 110 °C for 6 hours and finally the pyrolysed material was sieved over a 100 micron sieve to remove the larger particles. Yield: 11.6 grams of black powder. Figure 1 shows a SEM image of the obtained material.

### Example 2 (Comparative, in accordance with WO-A-2013/144879)

100.1 grams of a sieve fraction (particles smaller than 70 µm) of cellulose spheres (Cellets 90, HARKE Pharma, size 60-125 µm) were placed in a 1000 ml round bottom flask and were loaded with holmium nitrate via vacuum impregnation. To this end the cellulose spheres were impregnated via vacuum impregnation with an aqueous solution of holmium nitrate pentahydrate (19 g Ho(NO₃)₃·5H₂O, Sigma-Aldrich, 99.9 % purity, in 50 ml H₂O) in 5 minutes via a nozzle. The impregnated cellulose spheres were then dried at room temperature After 2 hours the flask was heated in an oil bath of 40 °C and further drying was done for another 7 hours to constant weight. This yielded 119 gram light pinkish powder. The resulting material was sieved over a 100 µm sieve and yielded 100 grams of a light pinkish powder. 30 g of this material was then heated in a further drying step in a nitrogen flow of 21 ml/h while heating to 110 °C for 25 hours. Subsequently, the material was heated to 300 °C (ramp 2 °C/min, isotherm 1 hour) and then to 800 °C (ramp 2 °C/min, isotherm 1 hour) in a pyrolysis step, which resulted in a black powder. After the pyrolysis step was completed, the material was allowed to cool to room temperature while the kept under a N₂ flow.

Finally the pyrolysed material was were treated with an excess demi-water with a few droplets of surfactant (Dreft soap for dish washing, which comprises a mixture of 5-15 % of anionic surfactants and < 5 % nonionic surfactants). The suspension was treated in an ultrasonic bath for 60 minutes and subsequently filtered, washed with 500 ml of demi-water and finally with 100 ml i-propanol. The pyrolysed material was dried in an oven at 110 °C for 6 hours and finally the pyrolysed material was sieved over a 100 micron sieve to remove the larger particles. Yield: 7.70 grams of black powder.

### Example 3 (Comparative)

30 grams of washed and dried ion exchange resin (Dowex 50WX4 H+ (*i.e.* a styrene-divinylbenzene polymer matrix functionalised with a sulphonic acid group), Fluka, 200-400 mesh) was added to 330 grams demi-H₂O and stirred at 750 rpm in a glass beaker. To this slurry 17.5 g of holmium nitrate pentahydrate (Ho(NO₃)₃ 5H₂O, Sigma-Aldrich, 99.9 % purity) was added and the mixture was stirred overnight, which resulted in the holmium cations being introduced into the ion exchange resin by ion exchange. Next, the slightly brownish particles were filtered and washed with 300 ml H₂O and 200 ml isopropanol (technical grade, VWR). 5 grams of this material were slurried in a 300 ml solution of Na₃PO₄ (3.75 g Na₃PO₄, Sigma-Aldrich, 98 % purity in 300 ml demi-water) and stirred at room temperature. After 3 hours the slurry was filtered and washed with 750 ml demi-water. Finally, the material was dried at 70 °C overnight yielding 5.37 grams of material.

### Analysis

ICP analyses were performed on samples of Examples 1-3 using a Thermo-Scientific iCAP 7000 series. Sample preparation was carried out by dissolving the particles in a concentrated HNO₃ solution (Lps, 65 % Pro Analysis (P.A.) in a microwave at 230 °C. The holmium content of the solutions (10 wt.% HNO₃ matrix) were then measured at 345 and 389 nm after calibration.

Carbon, nitrogen and sulphur (C, N, S) analyses of samples of Examples 1-3 was performed on a EuroVector Euro EA elemental analyser with additional vanadium pentoxide added to the samples to assure complete oxidation of the samples.

Powder X-ray diffraction (XRD) patterns of samples of Examples 1-3 were obtained with a Bruker D8 ADVANCE (Detector: SOL'X, Anode: Copper, wavelength: 1.542 Å, Primary Soller slit: 4°, Secundary Soller slit: 4°, Detector slit: 0.2 mm, Spinner: 15 RPM, Divergence slit: variable V20, Antiscatter slit: variable V20, Start: 10° 2 theta, Stop: 100° 2 theta, Stepsize: 0.05° 2 theta, Time/step: 8 sec, Sample preparation: Front loading).

The analysis results of Examples 1-3 are shown in Table 1 below.

**Table 1**

| Example No. | Holmium content (wt.%) | Carbon content (wt.%) | Nitrogen content (wt.%) | Sulphur content (wt.%) | XRD |
|---|---|---|---|---|---|
| 1 | 22.3 | 55.6 | 0.2 | 9.5 | Amorphous |
| 2 | 21.1 | 68.7 | 0.6 | < 0.05 | Amorphous |
| 3 | 22.0 | 68.0 | 0.7 | 9.5 | Amorphous |

### Leaching Test under neutral and acidic conditions

1.00 gram of the material according to Examples 1-3 was immersed in a 25 ml demi-water (pH neutral *i.e.* pH = 7), 3.1 wt.% HNO₃ solution (pH was 0.55 at room temperature) and 10 wt.% HNO₃ solution (pH was 0.11 at room temperature). After 2 h stirring at room temperature at 400 rpm, the material was filtered using a filtering apparatus with Whatman 589/5 filter paper. The holmium content of the filtrate was then analysed by ICP according to the method as described herein above. The results of the leaching experiments for Examples 1-3 , with the measured amount of holmium content of the filtrate in ppm by weight are the non-bracketed values shown in Table 2 below. The values shown in brackets Table 2 below is the holmium content of the filtrate in weight percent, based upon the total amount of holmium present in the 1.00 gram material of Examples 1-3, respectively.

**Table 2**

| | Ho content in ppm (wt.%) | | |
|---|---|---|---|
| Solution | Example 1 | Example 2 | Example 3 |
| Demi-water pH = 7 | < 1 (< 0.01) | < 1 (< 0.01) | 15 (0.17) |
| 3.1 wt.% HNO₃ | 3.7 (0.04) | 93 (1.10) | 1526 (17.11) |
| 10 wt.% HNO₃ | 3.6 (0.04) | 91 (1.08) | 3796 (42.56) |

### Example 4 (SiO₂ Coating of Ion-Exchange Resin-Spheres)

10 grams of washed and dried ion exchange resin (Dowex 50WX4 H+ (*i.e.* a styrene-divinylbenzene polymer matrix functionalised with a sulphonic acid group), Fluka, 200-400 mesh) spherically shaped particles, having a diameter between 34 and 74 microns was dispersed in a mixture of ethanol and water (96 ml deionised water, 613 ml ethanol). After addition of a surfactant solution (4 ml, 3.8 wt.% Lutensol®, AO5 in deionised water) the mixture was stirred for 30 minutes at room temperature and standard ambient pressure (*i.e.* 100 kPa). Afterwards, ammonia solution (3.2 ml, 32 wt.%) and an ethanolic tetraethyl orthosilicate solution were added (3.7 ml in 30 ml ethanol) together, which resulted in the hydrolysis/condensation reaction of the TEOS (tetraethyl orthosilicate) and the coating of the spheres with SiO₂. After stirring for 18 h at room temperature and standard ambient pressure (*i.e.* 100 kPa) the material was separated, washed two times with water and ethanol (50 ml each). Finally, the material was dried in a drying oven at 60 °C for 24 hours yielding an off-white powder.

### Example 5 (Pyrolysed, SiO₂ coated holmium containing spheres)

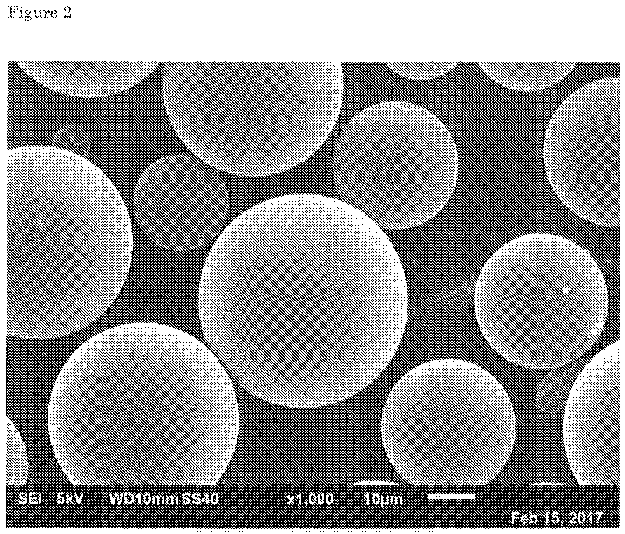

50.02 grams of material comparable to Example 4 was loaded with holmium by adding 18.65 grams of holmium nitrate pentahydrate (Ho(NO₃)₃ 5H₂O, Sigma-Aldrich, 99.9 % purity) to a 400 ml aqueous slurry of this material and stirring the slurry overnight. Next, the slightly brownish particles were filtered and washed with 500 ml demi-H₂O and 500 ml isopropanol (technical grade, VWR), respectively. Drying was performed in an oven at 105 °C overnight. This yielded 53.85 grams of a slightly brownish powder. This holmium loaded, SiO₂ coated material was heated in a pyrolysis step in a fluidised bed at 800 °C for 1 hour. After cooling and air stabilization, the black material was subsequently washed and dried. This yielded 29.77 grams of a black powder that contained 18.7 wt.% of holmium as determined by ICP analyses. C = 49.1 wt.%, N = 0.5 wt.%, S = 7.9 wt.%. Figure 2 shows a SEM image of the material and table 3 shows the elemental composition of the particles as found by SEM-EDS.

### Example 6 (partial calcination of pyrolysed, SiO₂ coated holmium containing spheres).

13.49 grams of a sieve fraction (< 60 micron) of the holmium loaded, SiO₂ coated material of Example 5 was heated in a fluidised bed at 455 °C for 10 hours in an air flow (31 nl/h). After cooling down the reactor, washing in water and isopropanol subsequently and drying in an oven at 110 °C overnight, 5.03 grams of a black material was isolated that contained 41.8 wt.% of holmium as determined by ICP analyses. Table 4 shows the elemental composition of the particles as found by SEM-EDS.

### Example 7 (SiO₂ coating of holmium containing carbon spheres)

1.0 gram of the material from Example 1 was dispersed in a mixture of ethanol and water (24 ml deionised water, 150 ml ethanol). After addition of a surfactant (72.5 mg, cetyltrimethylammoniumbromide, 95 %, Sigma-Aldrich) the mixture was stirred for 60 minutes at room temperature and standard ambient pressure (*i.e.* 100 kPa). Afterwards, ammonia solution (0.3 g, 32 wt.%) and an ethanolic tetraethyl orthosilicate solution were added (1.5 ml in 2.5 ml ethanol). After stirring for 18 h at room temperature and standard ambient pressure (*i.e.* 100 kPa) the material was separated, washed two times with water and ethanol (50 ml each). Finally, the material was dried in a drying oven at 60 °C for 24 h. Yield: 1.3 gram of a black powder. SEM-EDS elemental analyses of the sample revealed the presence of SiO₂ on the outside of the particles.

### Example 8 (multiple holmium addition steps)

An aqueous solution of holmium nitrate (4.50 gram of Ho(NO₃)₃ ·5H₂O in 18.02 g demi-H₂O) was added dropwise to 20,85 grams of oven dried, holmium loaded ion exchange resin (Dowex 50WX4 H+) particles (similar to example 1 before pyrolysis). The resulting material was dried overnight at 110 °C. The next day, 22.88 grams of the pinkish material was pyrolised at 800 °C under a nitrogen flow of 62 nl/h for 1 hour. After cooling and air stabilisation, 15.17 grams of a black powder was isolated that contained 33.8 wt.% of holmium by ICP analyses. C = 41.2 wt.%, N = 0.2 wt.%, S = 10.9 wt.%.

### Example 9 (monosized holmium loaded carbonized spheres)

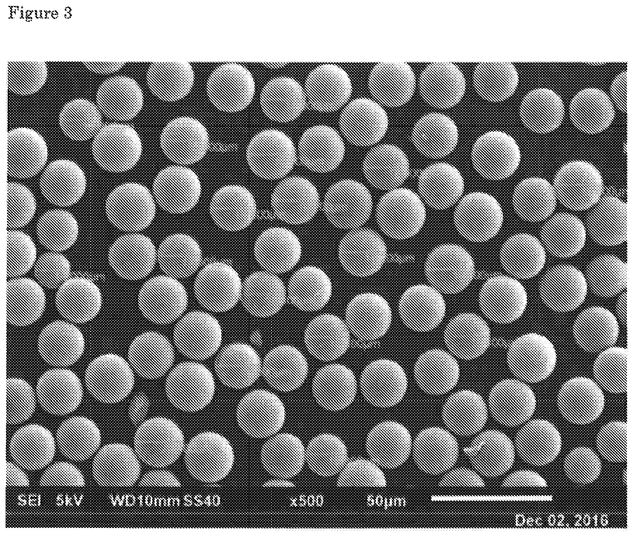

67.12 g of a suspension of Source30 (GE Healthcare Life Sciences) was placed in a beaker glass and dried in an oven at 110 °C overnight which resulted in 10.65 grams of material. To this was added dropwise a solution of holmium nitrate (16.42 grams of Ho(NO₃)₃·5H₂O in 17.56 g demi-H₂O). The wet material was placed in an oven and was dried at 110 °C overnight which resulted in a pinkish powder (23.63 grams). A portion of this powder (16.15 grams) was pyrolysed at 800 °C in a nitrogen flow for 1 hour. After cooling and air stabilisation, and subsequent washing with water and isopropanol, 6.11 grams of a black powder was isolated that contained 54.8 wt.% of holmium by ICP analyses. C = 27.8 wt.%, N = 0.7 wt.%, S = 0.7 wt.%. Figure 3 shows a SEM image of the material.

### Example 10 (cellulose based, holmium loaded carbonized particles)

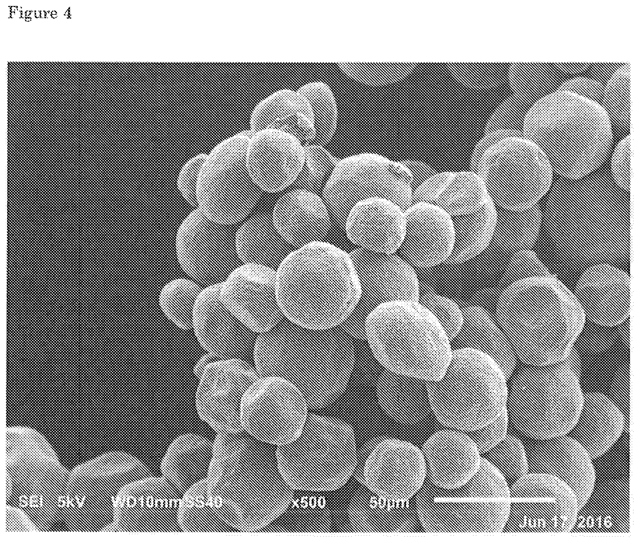

Material based on sulphur containing spherical cellulose was prepared according to a procedure similar to example 9, except now 40 ml of a suspension of Cellufine Max S-h (AMS Biotechnology) was used. Ho = 21.4 wt.% (ICP analysis); C = 64.0 wt.%, N = 1.0 wt.%, S = 0.8 wt.%. XRD indicated the material to be completely amorphous. Figure 4 shows a SEM image of the material.

### Example 11 (addition of hydrophylic surface groups)

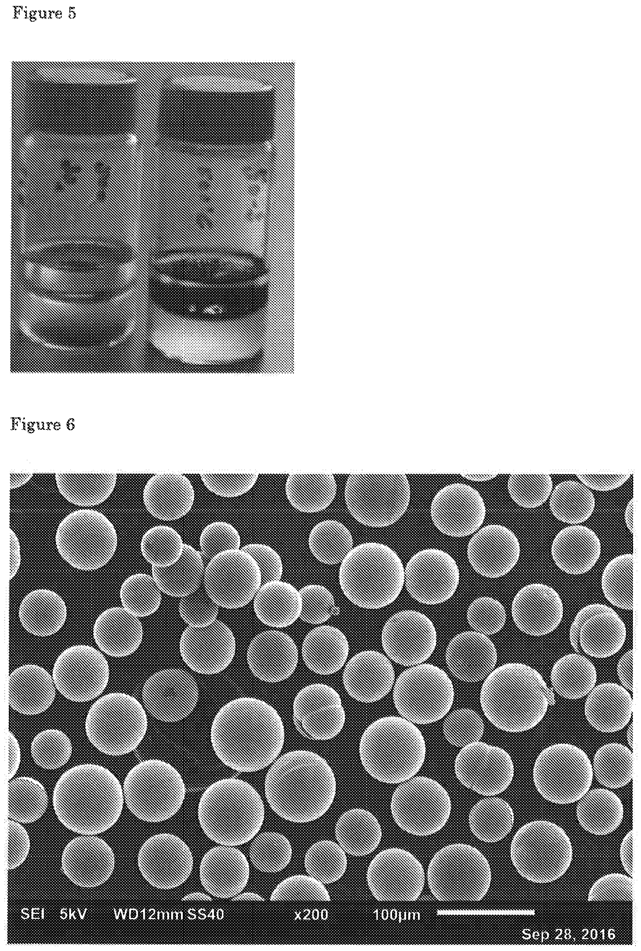

To 1.0 gram of carbonized holmium containing spheres of example 1 was added a solution of concentrated H₂SO₄ (5 ml, 96 %) at room temperature. The slurry was heated to 150 °C overnight without stirring. The next day, after cooling to room temperature, the black powder was washed with an excess of demi-water and was dried in an oven at 110 °C for 8 hours. This H₂SO₄ treated material appeared to be much more hydrophilic compared to the parent material of example 1, indicated by the distribution of the samples in a water-toluene biphasic system. This is demonstrated in figure 5, which shows the distribution of particles in a water-toluene biphasic system. Left: Example 11; all material is in water phase; Right: Example 1; all material is in toluene phase at the interface between water and toluene.

### Example 12 (SiO₂ coating of resin spheres, alternative procedure to Example 4)

10 grams of washed and dried ion exchange resin (Dowex 50WX4 H+ (*i.e.* a styrene-divinylbenzene polymer matrix functionalised with a sulphonic acid group), Fluka, 200-400 mesh) spherically shaped particles, having a diameter between 34 and 74 microns was dispersed in a mixture of ethanol and water (96 ml deionised water, 460 ml ethanol). After addition of cetyltrimethylammoniumbromide (75 mg, 95 % purity, Sigma-Aldrich) the mixture was stirred for 1 hour at room temperature and standard ambient pressure (*i.e.* 100 kPa). Afterwards, ammonia solution (3.2 ml, 32 wt.%) was added and stirred for another 15 minutes. Subsequently, an ethanolic tetraethyl orthosilicate solution was added (6.3 ml in 75 ml ethanol) together, which resulted in the hydrolysis/condensation reaction of the TEOS (tetraethyl orthosilicate) and the coating of the spheres with SiO₂. After stirring for 18 h at room temperature and standard ambient pressure (*i.e.* 100 kPa) the material was separated, washed two times with water and ethanol (50 ml each). Finally, the material was dried in a drying oven at 60 °C for 24 hours yielding an off-white powder. The material was analysed by SEM-EDS and the elemental composition is found in table 5.

### Example 13 (SiO₂ coating of holmium containing resin spheres)

The ion-exchange resin was loaded with holmium according to the procedure in Example 1 (stopped after drying in oven). 10 grams this sample were dispersed in a mixture of ethanol and water (96 ml deionised water, 613 ml ethanol). After addition of a surfactant solution (4 ml, 3.8 wt.% Lutensol® AO5 in deionised water) the mixture was stirred for 30 minutes at room temperature and standard ambient pressure (*i.e.* 100 kPa). Afterwards, ammonia solution (3.8 ml, 32 wt.%) and an ethanolic tetraethyl orthosilicate solution were added (6.7 ml in 100 ml ethanol) together, which resulted in the hydrolysis/condensation reaction of the TEOS (tetraethyl orthosilicate) and the coating of the spheres with SiO₂. After stirring for 18 h at room temperature and standard ambient pressure (*i.e.* 100 kPa) the material was separated, washed two times with water and ethanol (50 ml each). Finally, the material was dried in a drying oven at 60 °C for 24 hours yielding an off-white powder. After pyrolysis of this material at 800 °C in N₂ a black powder could be isolated that was analysed by SEM-EDS. The elemental composition of the carbonised material can be found in table 6 and a SEM image of the material can be found in figure 6.

### Example 14 (holmium containing resin spheres coated with PFA and SiO₂)

The ion-exchange resin was loaded with holmium according to the procedure in Example 1 (stopped after drying in oven). 10 g of this sample were mixed with furfuryl alcohol (12 g, purity 98 %, Sigma-Aldrich) and stirred for 3 hours. After separation, the spheres were washed with ethanol and immediately redispersed in a mixture of ethanol and water (96 ml deionised water, 460 ml ethanol). After addition of cetyltrimethylammoniumbromide (75 mg, 95 % purity, Sigma-Aldrich) the mixture was stirred for 1 hour at room temperature and standard ambient pressure (*i.e.* 100 kPa). Afterwards, ammonia solution (3.2 ml, 32 wt.%) was added and stirred for another 15 minutes. Subsequently, an ethanolic tetraethyl orthosilicate solution was added (6.3 ml in 75 ml ethanol) together, which resulted in the hydrolysis/condensation reaction of the TEOS (tetraethyl orthosilicate) and the coating of the spheres with SiO₂. After stirring for 18 h at room temperature and standard ambient pressure (*i.e.* 100 kPa) the material was separated, washed two times with water and ethanol (50 ml each). Finally, the material was dried in a drying oven at 60 °C for 24 hours yielding a greyish powder. After pyrolysis of this material at 800 °C in N₂, a black powder could be isolated that was analysed by SEM. The elemental composition by SEM-EDS of the carbonised material can be found in table 7.

### SEM Analysis

Examples 1, 5, 6, 9, 10, 12, 13 and 14 were analysed by SEM which was carried out with a Phenom ProX, Co. Phenom-World B.V. scanning electron microscope equipped with a specifically designed EDS detector to determine elemental composition. Figures 1, 2, 3, 4 and 6 show SEM images of examples 1, 5, 9, 10 and 13, respectively.

Example 5, 6, 12, 13 and 14 were analysed using energy dispersive X-ray spectroscopy (EDS) to determine the elemental composition. The results of this analysis is shown in Tables 3, 4, 5, 6 and 7, respectively.

**Table 3: SEM/EDS measurements of Example 5.**

| Element Symbol | Weight Conc. | Atomic Conc. |
|---|---|---|
| C | 49.7 | 74.3 |
| O | 15.4 | 17.2 |
| Si | 3.3 | 2.1 |
| S | 6.5 | 3.7 |
| Ho | 25.1 | 2.7 |

**Table 4: SEM/EDS measurements of Example 6.**

| Element Symbol | Weight Conc. | Atomic Conc. |
|---|---|---|
| C | 14.7 | 30.0 |
| O | 33.8 | 51.9 |
| Si | 12.3 | 10.8 |
| S | 2.4 | 1.8 |
| Ho | 36.8 | 5.5 |

**Table 5: SEM/EDS measurements of Example 12.**

| Element Symbol | Weight Conc. | Atomic Conc. |
|---|---|---|
| C | 45.5 | 54.8 |
| N | 14.0 | 14.4 |
| O | 27.5 | 24.8 |
| Si | 2.1 | 1.1 |
| S | 10.9 | 4.9 |

**Table 6: SEM/EDS measurements of Example 13.**

| Element Symbol | Weight Conc. | Atomic Conc. |
|---|---|---|
| C | 42.4 | 61.8 |
| Ho | 19.7 | 2.1 |
| O | 26.9 | 29.4 |
| Si | 8.1 | 5.1 |
| S | 2.9 | 1.6 |

**Table 7: SEM/EDS measurements of Example 14.**

| Element Symbol | Weight Conc. | Atomic Conc. |
|---|---|---|
| C | 32.4 | 50.6 |
| Ho | 20.7 | 2.4 |
| O | 31.7 | 37.1 |
| Si | 11.6 | 7.8 |
| S | 3.7 | 2.2 |

### Example 15 (Neutron activation)

The material of examples 1, 5, 6 and 9 was activated by neutron irradiation. For Examples 1 and 5 sieve fractions of < 50 µm were used. Irradiations were performed in the pneumatic rabbit system (PRS) in the reactor facilities in Delft, The Netherlands. The PRS (neutron flux of 5.10¹² cm⁻²s⁻¹) irradiations were carried out on samples of *ca*. 200 mg, which were packed in polyethylene vials. The irradiation time was 10 hours. The 10 hours irradiation resulted in an estimated relatively high activity depending on the holmium content and gives an amount of microsphere associated radioactivity needed for the treatment of a patient, including transport and logistics to the patient. A typical amount of Dose (Gy) and activity (MBq) of holmium-166 treatment can be calculated: examples of amounts of activity for typical liver weights are given for a liver absorbed dose of 60 Gy, which equates to 3.8 GBq/kg (liver weight). A standard liver will have a weight of around 1.5 kg.

The raw data of the neutron activation and results of free holmium measurements are shown in table 8. These data were collected using a Perkin-Elmer Wizard III Wallac, gamma-counter at the facility in Delft.

**Table 8**

| Example | Time | Ho-166 Counts | Ho-166 CPM | Result (%) |
|---|---|---|---|---|
| Ex. 1 | 600.05 | 342554.67 | 34897.75 | 100.0 |
| Ex. 5 | 600.06 | 262926.33 | 26663.01 | 100.0 |
| Ex. 6 | 600.02 | 388931 | 39786.87 | 100.0 |
| Ex. 9 | 600.03 | 224128.33 | 22789.75 | 100.0 |
| Ex. 1 | 600.04 | 405 | 40.5 | 0.0 |
| Ex. 5 | 600.04 | 227 | 22.7 | 0.0 |
| Ex. 6 | 600.04 | 12074 | 1208.29 | 0.6 |
| Ex. 9 | 600.04 | 943 | 94.3 | 0.1 |

Material properties after 10 hours of neutron irradiation are summarised in Table 9, the activity of the material is shown in Table 10.

**Table 9**

| | | | size measurement | visual | free holmium |
|---|---|---|---|---|---|
| example | weight (mg) | holmium content (%) | mean (µm) | result | result < 1 % |
| Ex. 1 | 203.54 | 26.9 | 29.33 | passed | 0.023 |
| Ex. 5 | 203.38 | 18.7 | 28.09 | passed | 0.017 |
| Ex. 6 | 207.85 | 41.8 | 35.95 | some artefacts | 0.607 |
| Ex. 9 | 203.04 | 54.8 | 61.94 | passed | 0.083 |

**Table 10**

| example | reactor position | time irradiated | pre-calculated (GBq) | measured activity (GBq) e.o.b. | Δ GBq |
|---|---|---|---|---|---|
| Ex. 1 | BP3 | 10 hrs | 14 | 14.3 | + 0.3 |
| Ex. 5 | BP3 | 10 hrs | 9.7 | 9.1 | - 0.6 |
| Ex. 6 | BP3 | 10 hrs | 22.3 | 22.6 | + 0.3 |
| Ex. 9 | BP3 | 10 hrs | 28.4 | 23.9 | - 4.5 |

The typical clinical amount of activity is *ca.* 6 GBq in 600 mg of microspheres. End of bombardment (e.o.b.) activity is twice the activity needed for patient treatment = 12 GBq.

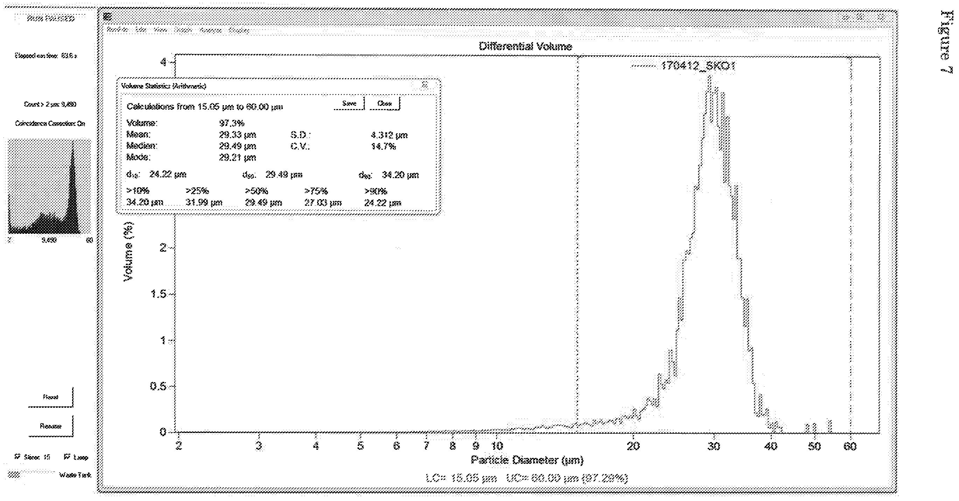

Figure 7 shows the particle size distribution of Example 1 material (sieve fraction of < 50 µm) after neutron activation as determined with Multisizer volume distribution analysis. The mean is 29.33 µm, the distribution 97.3 % (range 15-60 µm).

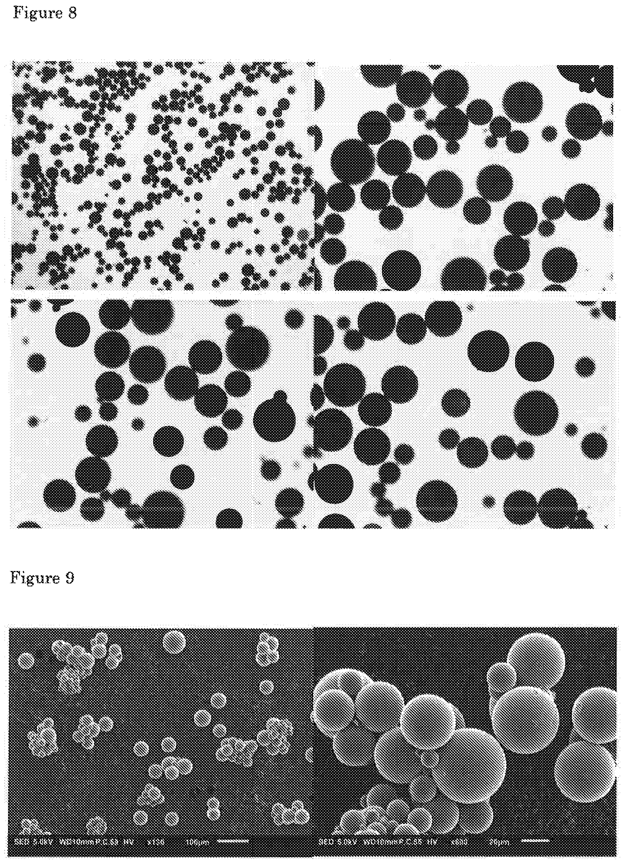

Figure 8 shows light microscopic graphs of neutron activated microspheres (Example 1; sieve fraction of < 50 µm), (top left: 100 × magnification; top right: 400 × magnification; bottom left: 400 × magnification; bottom right: 400 × magnification).

Figure 9 shows scanning electron microscope images of Example 1 material (sieve fraction of < 50 µm) after neutron activation. The particles are perfectly round and suitable for clinical use.

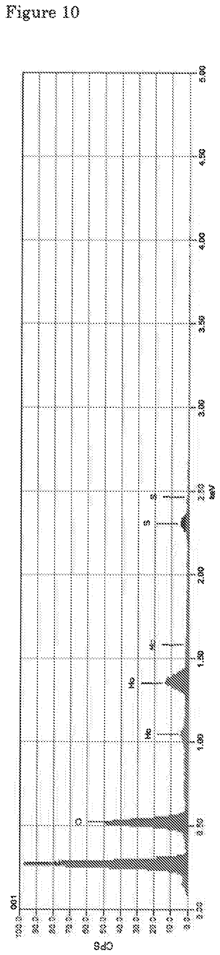

Figure 10 shows an EDS analysis of Example 1 material (sieve fraction of < 50 µm) after neutron activation, identification of compounds on the surface.

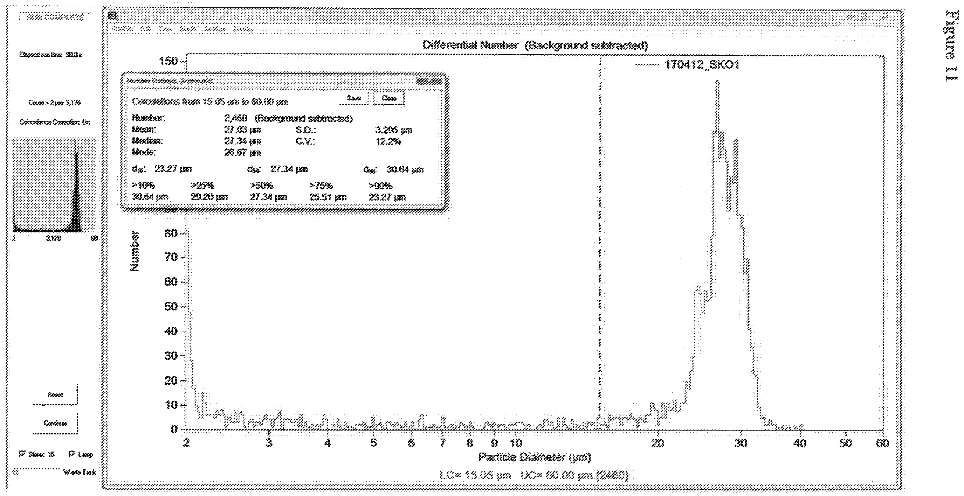

Figure 11 shows the particle size distribution of Example 5 material (sieve fraction of < 50 µm) after neutron activation as determined with Multisizer volume distribution analysis. The mean is 28.09 µm, the distribution 99.6 % (range 15-60 µm, using volume statistics). The values mentioned in the figure express number statistics, rather than volume statistics.

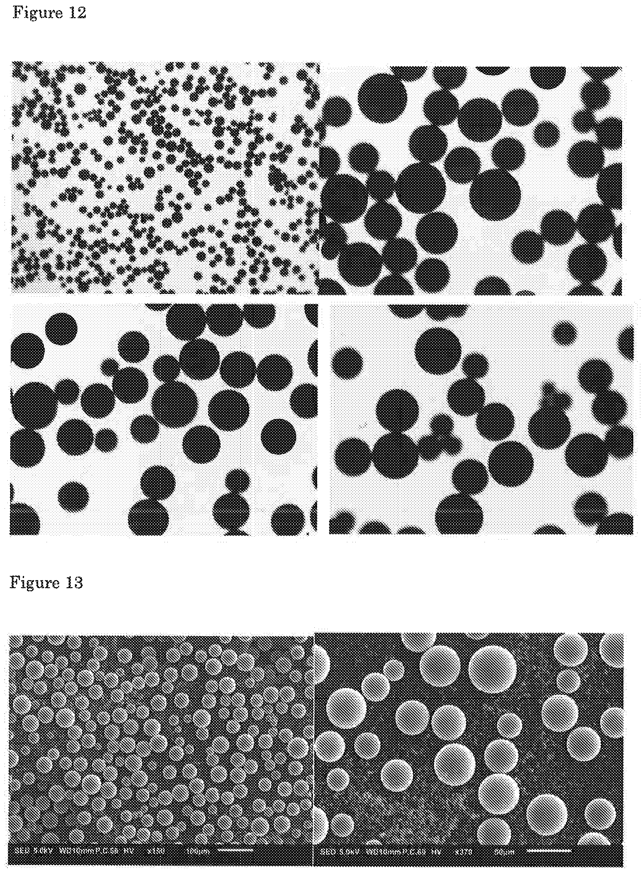

Figure 12 shows light microscopic graphs of neutron activated microspheres (Example 5; sieve fraction of < 50 µm), (top left: 100 × magnification; top right: 400 × magnification; bottom left: 400 × magnification; bottom right: 400 × magnification).

Figure 13 shows scanning electron microscope images of Example 5 material (sieve fraction of < 50 µm) after neutron activation. The particles are perfectly round and suitable for clinical use.

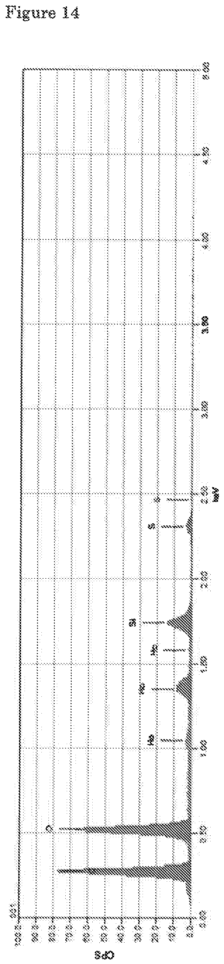

Figure 14 shows an EDS analysis of Example 5 material (sieve fraction of
< 50 µm) after neutron activation, identification of compounds on the surface.

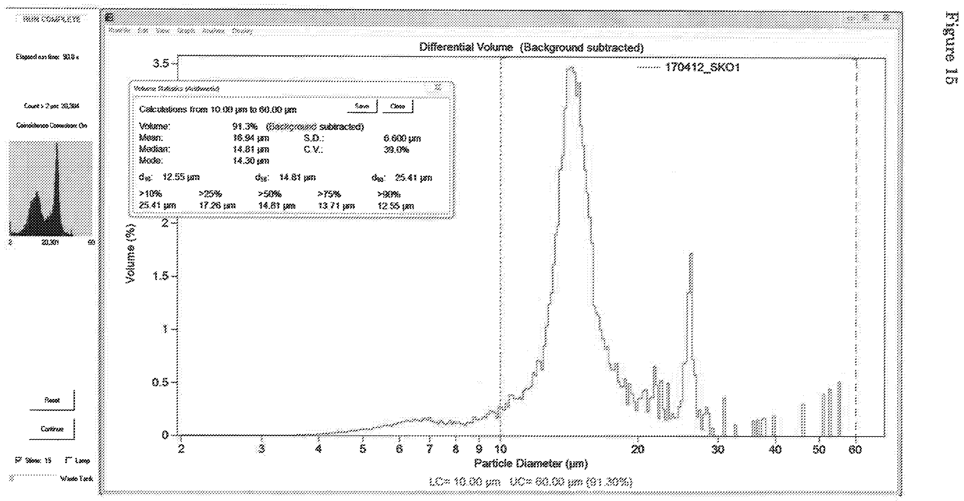

Figure 15 shows the particle size distribution of Example 9 material after neutron activation as determined with Multisizer volume distribution analysis. The mean is 16.94 µm, the distribution 91.3 % (range 10-60 µm).

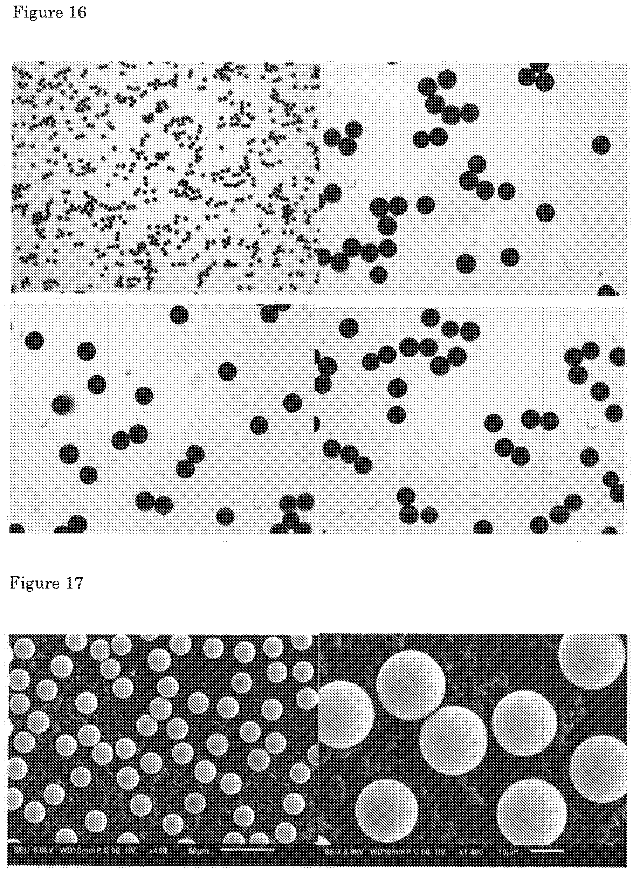

Figure 16 shows light microscopic graphs of neutron activated microspheres (Example 9), (top left: 100 × magnification; top right: 400 × magnification; bottom left: 400 × magnification; bottom right: 400 × magnification).

Figure 17 shows scanning electron microscope images of Example 9 material after neutron activation. The particles are perfectly round and suitable for clinical use.

## Claims

1. Body comprising an oxide of lanthanide, preferably in the form of particles, supported on a sulphur containing carbon based particle, wherein said body comprises lanthanide to sulphur in an atomic ratio ranging from 1 : 0.01 to 1 : 10,
for use as
- a medicament, preferably for treating a cancer, in particular a cancer of the brain, pancreas, lymph, lung, head and neck, prostate, breast, liver, intestines, thyroid, stomach, or kidney; and/or
- a diagnostic agent, preferably for detecting a cancer, in particular a cancer of the brain, pancreas, lymph, lung, head and neck, prostate, breast, liver, intestines, thyroid, stomach, or kidney.

2. Body for use according to claim 1, wherein said use is in surgery, therapy and/or *in vivo* diagnostics, wherein the surgery, therapy and/or *in vivo* diagnostics preferably comprises:
- imaging, preferably selected from the list consisting of magnetic resonance imaging, nuclear scanning imaging, X-ray imaging, positron emission tomography (PET) imaging, single-photon emission computed tomography (SPECT) imaging, X-ray computed tomography (CT) imaging, scintigraphy imaging, ultrasound, fluorescent imaging and combinations thereof;
- drug delivery;
- cellular labeling; and/or,
- radiotherapy, preferably radio-embolisation,
wherein the surgery, therapy and/or *in vivo* diagnostics is preferably a method for detection and/or treatment of a cancer, in particular a cancer of the brain, pancreas, lymph, lung, head and neck, prostate, breast, liver, intestines, thyroid, stomach, or kidney.

3. Body for use according to claim 1 or 2,
wherein the body is administered as a medicament by catheter, in a method of surgery, therapy and/or *in vivo* diagnostics; by injection, preferably intravenous or direct injection; infusion; or via a skin patch.

4. Body for use according to any one of claims 1-3, wherein said body comprises
- lanthanide and sulphur in a total amount of at least 5 wt.%, preferably at least 10 wt.%, more preferably at least 20 wt.%, more preferably at least 30 wt.%, even more preferably at least 40 wt.%, and in particular at least 50 wt.%, and in a total amount of at most 90 wt.%, preferably at most 80 wt.%, preferably at most 70 wt.%, more preferably at most 60 wt.%, calculated as the total amount of elemental lanthanide and elemental sulphur based on the weight of said body; and/or
- elemental carbon in an amount of 5-80 wt.%, preferably 10-80 wt.%, more preferably 20-70 wt.% and even more preferably 40-70 wt.%, based on the weight of said body, wherein said elemental carbon is preferably in the form of amorphous carbon, graphitic carbon or combinations thereof.

5. Body for use according to any one of claims 1-4, wherein said carbon based particle further comprises a carbon source material selected from the group consisting of a polymeric matrix; cellulose; cellulose-like material; carbohydrate; active carbon; and combinations thereof,
preferably said carbon source material further comprises at least one sulphur group.

6. Body for use according to any one of claims 1-5, wherein said lanthanide at least partially comprises a radioactive isotope of said lanthanide.

7. Body for use according to any one of claims 1-6, wherein said lanthanide is holmium.

8. Body for use according to any one of claims 1-7, wherein said body has a diameter of
- 0.1 to 500 µm, preferably 1-400 µm, more preferably 1-300 µm and even more preferably 1-200 µm, and in particular 15-60 µm; or
- 1 to 100 µm, preferably 1-50 µm, more preferably 1-30 µm and even more preferably 5-20 µm; or
- 0.01 to 0.50 µm, and preferably 0.05 to 0.30 µm.

9. Body for use according to one of claims 1-8, wherein said body has
- a sphericity of more than 0.75, preferably more than 0.85 and more preferably is at least 0.95; and/or
- a density of > 0.8 g/ml to 8.0 g/ml, preferably about 0.9-6.0 g/ml, more preferably 1.0-4.0 g/ml, more preferably 1.0-3.5 g/ml, more preferably 1.05-2.00 g/ml, and more preferably 1.1-1.6 g/ml.

10. Body for use according to any one of claims 1-9, wherein said body further comprises
- on its surface one or more functional groups, preferably said one or more functional groups comprise hydrophilic groups and/or active groups; and/or
- other elements selected from the group consisting of iron, gadolinium, manganese, phosphorous, iodine, iridium, rhenium and combinations thereof.

11. Body for use according to any one of claims 1-10, wherein the body, in particular the oxide of lanthanide of the body, are at least partly coated by a layer of an element or by an oxide of an element, wherein said element is selected from the group consisting of silicon, titanium, zirconium, hafnium, cerium, aluminium, niobium, tantalum and combinations thereof.

12. Suspension comprising a number of bodies as defined in any one of claims 1-11, said suspension being a therapeutic suspension, an MRI scanning suspension, and/or a nuclear scanning suspension.

13. Use of a body as defined in any one of claims 1-12 as contrast agent, such as blood pool contrast agent, for computer tomogram (CT) including dual energy CT, or magnetic resonance imaging (MRI).

14. Pharmaceutical composition comprising
- a body comprising an oxide of lanthanide, supported on a sulphur containing carbon based particle, wherein said body comprises lanthanide to sulphur in an atomic ratio ranging from 1 : 0.01 to 1 : 10, and
- a pharmaceutically acceptable carrier, diluent and/or excipient.

## Patentansprüche

1. Körper, umfassend ein Lanthanoidoxid, vorzugsweise in der Form von Partikeln, getragen auf einem schwefelhaltigen auf Kohlenstoff-basierenden Partikel, wobei der Körper Lanthanid zu Schwefel in einem Atomverhältnis im Bereich von 1: 0,01 bis 1: 10 umfasst, zur Verwendung als
- ein Medikament, vorzugsweise zum Behandeln eines Krebses, insbesondere eines Krebses des Gehirns, der Bauchspeicheldrüse, der Lymphe, der Lunge, des Kopfes und des Halses, der Prostata, der Brust, der Leber, des Darms, der Schilddrüse, des Magens, oder der Niere; und/oder
- ein diagnostisches Mittel, vorzugsweise zum Detektieren eines Krebses, insbesondere eines Krebses des Gehirns, der Bauchspeicheldrüse, der Lymphe, der Lunge, des Kopfes und des Halses, der Prostata, der Brust, der Leber, des Darms, der Schilddrüse, des Magens oder der Niere.

2. Körper zur Verwendung nach Anspruch 1, wobei die Verwendung in Chirurgie, Therapie und/oder in-vivo-Diagnostik ist,
wobei die Chirurgie, Therapie und/oder in-vivo-Diagnostik vorzugsweise umfasst:
- Bildgebung, vorzugsweise ausgewählt aus der Liste bestehend aus Magnetresonanztomographie, Kernabtastbildgebung, Röntgenbildgebung, Positronenemissionstomographie (PET), Einzelphotonenemissions-Computertomographie (SPECT), Röntgen-Computertomographie (CT) Bildgebung, Szintigraphie-Bildgebung, Ultraschall, Fluoreszenzbildgebung und Kombinationen davon;
- Arzneimittelabgabe;
- zelluläre Markierung; und/oder,
- Strahlentherapie, vorzugsweise Radioembolisation, wobei die Chirurgie, Therapie und/oder in-vivo-Diagnostik vorzugsweise ein Verfahren zur Detektion und/oder Behandlung eines Krebses ist, insbesondere eines Krebses des Gehirns, der Bauchspeicheldrüse, der Lymphe, der Lunge, des Kopfes und des Halses, der Prostata, der Brust, der Leber, des Darms, der Schilddrüse, des Magens oder der Niere.

3. Körper zur Verwendung nach Anspruch 1 oder 2, wobei der Körper als ein Medikament durch Katheter, in einem Verfahren der Chirurgie, Therapie und/oder in-vivo-Diagnostik verabreicht wird; durch Injektion, vorzugsweise intravenöse oder direkte Injektion; Infusion; oder über ein Hautpflaster.

4. Körper zur Verwendung nach einem der Ansprüche 1 - 3, wobei der Körper umfasst
- Lanthanoid und Schwefel in einer Gesamtmenge von wenigstens 5 Gew.-%, vorzugsweise wenigstens 10 Gew.-%, bevorzugter wenigstens 20 Gew.-%, bevorzugter wenigstens 30 Gew.-%, noch bevorzugter wenigstens 40 Gew.-%und insbesondere wenigstens 50 Gew.-%, und in einer Gesamtmenge von höchstens 90 Gew.-%, vorzugsweise höchstens 80 Gew.-%, vorzugsweise höchstens 70 Gew.-%, bevorzugter höchstens 60 Gew.-%, berechnet als die Gesamtmenge an elementarem Lanthanid und elementarem Schwefel basierend auf dem Gewicht des Körpers; und/oder
- elementaren Kohlenstoff in einer Menge von 5 - 80 Gew.-%, vorzugsweise 10 - 80 Gew.-%, bevorzugter 20 - 70 Gew.-% und noch bevorzugter 40 - 70 Gew.-%, basierend auf dem Gewicht des Körpers, wobei der elementare Kohlenstoff vorzugsweise in der Form von amorphem Kohlenstoff, graphitischem Kohlenstoff oder Kombinationen davon ist.

5. Körper zur Verwendung nach einem der Ansprüche 1 - 4, wobei das Kohlenstoff-basierende Partikel ferner ein Kohlenstoffquellenmaterial umfasst, ausgewählt aus der Gruppe bestehend aus einer Polymermatrix; Zellulose; zelluloseartigem Material; Kohlenhydrat; Aktivkohle; und Kombinationen davon, vorzugsweise das Kohlenstoffquellenmaterial ferner wenigstens eine Schwefelgruppe umfasst.

6. Körper zur Verwendung nach einem der Ansprüche 1 - 5, wobei das Lanthanid wenigstens teilweise ein radioaktives Isotop des Lanthanids umfasst.

7. Körper zur Verwendung nach einem der Ansprüche 1 - 6, wobei das Lanthanoid Holmium ist.

8. Körper zur Verwendung nach einem der Ansprüche 1 - 7, wobei der Körper einen Durchmesser hat von
- 0,1 bis 500 µm, vorzugsweise 1 - 400 µm, bevorzugter 1 - 300 pm und noch bevorzugter 1 - 200 µm, und insbesondere 15 - 60 µm; oder
- 1 bis 100 µm, vorzugsweise 1 - 50 µm, bevorzugter 1 - 30 µm und noch bevorzugter 5 - 20 µm; oder
- 0,01 bis 0,50 µm, und vorzugsweise 0,05 bis 0,30 µm.

9. Körper zur Verwendung nach einem der Ansprüche 1 - 8, wobei der Körper hat
- eine Sphärizität von mehr als 0,75, vorzugsweise mehr als 0,85 und bevorzugter wenigstens 0,95 ist; und/oder
- eine Dichte von > 0,8 g/ml bis 8,0 g/ml, vorzugsweise etwa 0,9-6,0 g/ml, bevorzugter 1,0-4,0 g/ml, bevorzugter 1,0-3,5 g/ml, bevorzugter 1,05-2,00 g/ml, und bevorzugter 1,1-1,6 g/ml.

10. Körper zur Verwendung nach einem der Ansprüche 1 - 9, wobei der Körper ferner umfasst
- auf seiner Oberfläche eine oder mehrere funktionelle Gruppen, vorzugsweise umfassen die eine oder mehreren funktionellen Gruppenhydrophile Gruppen und/oder aktive Gruppen; und/oder
- andere Elemente ausgewählt aus der Gruppe bestehend aus Eisen, Gadolinium, Mangan, Phosphor, Jod, Iridium, Rhenium und Kombinationen davon.

11. Körper zur Verwendung nach einem der Ansprüche 1 - 10, wobei der Körper, insbesondere das Lanthanoidoxid des Körpers, wenigstens teilweise durch eine Schicht eines Elements oder durch ein Oxid eines Elements beschichtet ist, wobei das Element ausgewählt ist aus der Gruppe bestehend aus Silizium, Titan, Zirkonium, Hafnium, Cer, Aluminium, Niob, Tantal und Kombinationen davon.

12. Suspension, umfassend eine Anzahl von Körpern, wie in einem der Ansprüche 1 - 11 definiert, wobei die Suspension eine therapeutische Suspension, eine MRT-Scansuspension, und/oder eine Kernscansuspension ist.

13. Verwendung eines Körpers nach einem der Ansprüche 1 - 12 als Kontrastmittel, wie Blutpool-Kontrastmittel, für Computertomogramm (CT) einschließlich Dual-Energy-CT, oder Magnetresonanztomographie (MRT).

14. Pharmazeutische Zusammensetzung, umfassend
- einen Körper, umfassend ein Lanthanoidoxid, getragen auf einem schwefelhaltigen Kohlenstoff-basierenden Partikel, wobei der Körper Lanthanid zu Schwefel in einem Atomverhältnis im Bereich von 1: 0,01 bis 1: 10 umfasst, und
- einen pharmazeutisch verträglichen Träger, Verdünnungsmittel und/oder Hilfsstoff.

## Revendications

1. Corps comprenant un oxyde de lanthanide, de préférence sous forme de particules, supporté sur une particule à base de carbone contenant du soufre, où ledit corps comprend du lanthanide au soufre dans un rapport atomique allant de 1:0,01 à 1:10,
destiné à être utilisé comme
- un médicament, de préférence pour traiter un cancer, en particulier un cancer du cerveau, du pancréas, de la lymphe, du poumon, de la tête et du cou, de la prostate, du sein, du foie, des intestins, de la thyroïde, de l'estomac ou du rein; et/ou
- un agent de diagnostic, de préférence pour détecter un cancer, en particulier un cancer du cerveau, du pancréas, de la lymphe, du poumon, de la tête et du cou, de la prostate, du sein, du foie, des intestins, de la thyroïde, de l'estomac ou du rein.

2. Corps destiné à être utilisé selon la revendication 1, où ladite utilisation est en chirurgie, thérapie et/ou diagnostic *in vivo,* où la chirurgie, la thérapie et/ou le diagnostic *in vivo* comprend de préférence:
- une imagerie, de préférence choisie dans la liste consistant en l'imagerie par résonance magnétique, l'imagerie par balayage nucléaire, l'imagerie aux rayons X, l'imagerie par tomographie par émission de positons (TEP), l'imagerie par tomographie par émission monophotonique (SPECT), l'imagerie par tomodensitométrie (TDM), l'imagerie par scintigraphie, l'échographie, l'imagerie par fluorescence et leurs combinaisons ;
- l'administration de médicaments ;
- le marquage cellulaire ; et/ou,
- la radiothérapie, de préférence la radio-embolisation,
où la chirurgie, la thérapie et/ou le diagnostic *in vivo* est de préférence un procédé pour la détection et/ou le traitement d'un cancer, en particulier un cancer du cerveau, du pancréas, de la lymphe, du poumon, de la tête et du cou, de la prostate, du sein, du foie, des intestins, de la thyroïde, de l'estomac ou du rein.

3. Corps destiné à être utilisé selon la revendication 1 ou 2, où le corps est administré sous forme d'un médicament par cathéter, dans un procédé de chirurgie, de thérapie et/ou de diagnostic *in vivo;* par injection, de préférence injection intraveineuse ou directe ; perfusion ; ou via un timbre cutané.

4. Corps destiné à être utilisé selon l'une quelconque des revendications 1-3, où ledit corps comprend
- un lanthanide et du soufre en une quantité totale d'au moins 5 % en poids, de préférence d'au moins 10 % en poids, de préférence encore d'au moins 20 % en poids, de préférence encore d'au moins 30 % en poids, de préférence encore d'au moins 40 % en poids, et en particulier d'au moins 50 % en poids, et en une quantité totale d'au plus 90 % en poids, de préférence d'au plus 80 % en poids, de préférence d'au plus 70 % en poids, de préférence encore d'au plus 60 % en poids, calculée comme la quantité totale de lanthanide élémentaire et de soufre élémentaire sur la base du poids dudit corps ; et/ou
- du carbone élémentaire en une quantité de 5-80 % en poids, de préférence de 10-80 % en poids, de préférence encore de 20-70 % en poids et de préférence encore de 40-70 % en poids, sur la base du poids dudit corps, où ledit carbone élémentaire est de préférence sous forme de carbone amorphe, de carbone graphitique ou de combinaisons de ceux-ci.

5. Corps destiné à être utilisé selon l'une quelconque des revendications 1-4, où ladite particule à base de carbone comprend en outre un matériau source de carbone choisi dans le groupe consistant en une matrice polymère ; la cellulose ; un matériau de type cellulose ; un glucide ; le carbone activé ; et leurs combinaisons,
de préférence ledit matériau source de carbone comprend en outre au moins un groupe soufre.

6. Corps destiné à être utilisé selon l'une quelconque des revendications 1-5, où ledit lanthanide comprend au moins partiellement un isotope radioactif dudit lanthanide.

7. Corps destiné à être utilisé selon l'une quelconque des revendications 1-6, où ledit lanthanide est l'holmium.

8. Corps destiné à être utilisé selon l'une quelconque des revendications 1-7, où ledit corps a un diamètre de
- 0,1 à 500 µm, de préférence 1-400 µm, de préférence encore 1-300 µm et de préférence encore 1-200 µm, et en particulier 15-60 µm ; ou
- 1 à 100 µm, de préférence 1-50 µm, de préférence encore 1-30 µm et de préférence encore 5-20 µm ; ou
- 0,01 à 0,50 µm, et de préférence 0,05 à 0,30 µm.

9. Corps destiné à être utilisé selon l'une des revendications 1-8, où ledit corps a
- une sphéricité de plus de 0,75, de préférence de plus de 0,85 et de préférence encore d'au moins 0,95 ; et/ou
- une masse volumique de > 0,8 g/ml à 8,0 g/ml, de préférence d'environ 0,9-6,0 g/ml, de préférence encore de 1,0-4,0 g/ml, de préférence encore de 1,0-3,5 g/ml, de préférence encore de 1,05-2,00 g/ml, et de préférence encore de 1,1-1,6 g/ml.

10. Corps destiné à être utilisé selon l'une quelconque des revendications 1-9, où ledit corps comprend en outre
- sur sa surface un ou plusieurs groupes fonctionnels, de préférence lesdits un ou plusieurs groupes fonctionnels comprennent des groupes hydrophiles et/ou des groupes actifs ; et/ou
- d'autres éléments choisis dans le groupe consistant en le fer, le gadolinium, le manganèse, le phosphore, l'iode, l'iridium, le rhénium et leurs combinaisons.

11. Corps destiné à être utilisé selon l'une quelconque des revendications 1-10, où le corps, en particulier l'oxyde de lanthanide du corps, est au moins partiellement revêtu d'une couche d'un élément ou d'un oxyde d'un élément, où ledit l'élément est choisi dans le groupe consistant en le silicium, le titane, le zirconium, le hafnium, le cérium, l'aluminium, le niobium, le tantale et leurs combinaisons.

12. Suspension comprenant un certain nombre de corps tels que définis dans l'une quelconque des revendications 1-11, ladite suspension étant une suspension thérapeutique, une suspension pour balayage IRM et/ou une suspension pour balayage nucléaire.

13. Utilisation d'un corps tel que défini dans l'une quelconque des revendications 1-12 comme agent de contraste, tel qu'un agent de contraste de pool sanguin, pour la tomodensitométrie (TDM) y compris la TDM à double énergie, ou l'imagerie par résonance magnétique (IRM).

14. Composition pharmaceutique comprenant
- un corps comprenant un oxyde de lanthanide, supporté sur une particule à base de carbone contenant du soufre, où ledit corps comprend du lanthanide au soufre dans un rapport atomique allant de 1:0,01 à 1:10, et
- un vecteur, diluant et/ou excipient pharmaceutiquement acceptable.
